(19) Europäisches Patentamt
European Patent Office
Office européen des brevets

(11) Veröffentlichungsnummer : **0 162 804**
**B1**

(12) # EUROPÄISCHE PATENTSCHRIFT

(45) Veröffentlichungstag der Patentschrift : **25.05.88**

(51) Int. Cl.⁴ : **C 07 D257/10**, C 07 F 15/00

(21) Anmeldenummer : **85810162.9**

(22) Anmeldetag : **12.04.85**

(54) **Substituierte Dibenzotetraaza-[14]-annulenmetallkomplexe, Verfahren zu ihrer Herstellung und ihre Verwendung.**

(30) Priorität : **18.04.84 CH 1949/84**

(43) Veröffentlichungstag der Anmeldung : **27.11.85 Patentblatt 85/48**

(45) Bekanntmachung des Hinweises auf die Patenterteilung : **25.05.88 Patentblatt 88/21**

(84) Benannte Vertragsstaaten : **CH DE FR GB LI NL**

(56) Entgegenhaltungen :
**EP-A- 0 030 879**
**EP-A- 0 047 198**
**US-A- 4 465 630**

(73) Patentinhaber : **CIBA-GEIGY AG**
**Klybeckstrasse 141**
**CH-4002 Basel (CH)**

(72) Erfinder : **Hunziker, Max, Dr.**
**Bösingenfeldstrasse 34**
**CH-3178 Bösingen (CH)**

**EP 0 162 804 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft in 2-, 3-, 7-, 11-, 12- und 16- Stellung substituierte Dibenzo [b,i] [1, 4, 8, 11] tetraaza — [14] — annulene, Verfahren zu ihrer Herstellung und ihre Verwendung in elektrooptischen und thermooptischen Anzeigeelementen, als Elektronendonatoren für elektrisch leitende Chargetransferkomplexe und als Färbemittel für farblose diskotische Flüssigkristalle.

Es ist bekannt, dass komplexbildente macrocyclische Verbindungen diskotische Mesophasen ausbilden können. In Mol. Cryst. Liq. Cryst. 1983, Vol. 100, S. 275 sind octasubstituierte Phthalocyanine beschrieben, die diskotische Mesophasen aufweisen. In J. Am. Chem. Soc., 1982, 104, S. 5245-5247 sind octasubstituierte Metallophthalocyamine mit ähnlichen Eigenschaften beschrieben. Aus Mol. Cryst. Liq. Cryst., Vol. 56, S. 303-309 (1980) ist es bekannt, dass Octan-dodecylester von Uroporphyrin eine flüssigkristalline Phase besitzen, die allerdings nur in einem sehr engen Bereich von 0,1 °C auftritt.

Metallkomplexe von substituierten Dibenzotetraaza — [14] — annulenen sind ebenfalls schon beschrieben worden, vgl. z. B. DE-A- 2 260 761, DE-A-2 214 336 und EP-A-0 073 456. Metallkomplexe mit diskotischen Mesophasen sind noch nicht beschrieben worden.

Gegenstand der Erfindung sind substituierte Dibenzotetraaza — [14] — annulenmetallkomplexe der Formel I

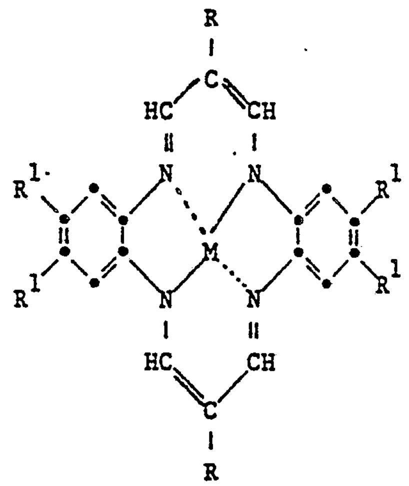

(I)

worin M für ein zweiwertiges Metallatom aus der Gruppe Eisen, Kobalt, Nickel, Kupfer, Palladium und Platin steht, R den Rest $H(CH_2)_m—$ bedeutet, worin m eine ganze Zahl von 4 bis 18 bedeutet, und $R^1$ für den Rest $H(CH_2)_n—X—$, $H(CH_2)_p—Y—CH_2—Z—$ oder $H(CH_2)_q—[—O—(CH_2)_r—]—_sZ—$ stehen, worin n eine ganze Zahl von 4 bis 18 bedeutet und X für eine direkte Bindung, —S—, —SO—, $—SO_2—$, —O—, —C(O)O— oder —O(O)C— steht, Y und Z unabhängig —O— oder —S— bedeuten, p für eine ganze Zahl von 1 bis 15 steht, r für eine ganze Zahl von 2 bis 6, s für eine ganze Zahl von 1 bis 6 und q für eine Zahl von 1 bis 19 $—(r + 1) \cdot s$ stehen.

In Formel I sind m und n unabhängig voneinander bevorzugt eine ganze Zahl von 6 bis 14, p bevorzugt eine Zahl von 3 bis 11 und die Gruppe $H—(CH_2)_q—[—O—(CH_2)_r—]—_sZ—$ weist bevorzugt 6 bis 14 Kettenglieder auf. Der Unterschied in der Kettenlänge der R— und $R^1$— Gruppen beträgt bevorzugt höchstens 6, besonders höchstens 4 Kettenglieder. Insbesondere beträgt dieser Unterschied 1 und 2 Kettenglieder oder die Zahl der Kettenglieder ist identisch.

X in Formel I bedeuten bevorzugt eine direkte Bindung, —S— und besonders —O—. Y und Z in Formel I stellen bevorzugt —O— dar. Beispiele für R und den Rest $H(CH_2)_n—$ als lineares Alkyl sind :

n-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl und n-Octadecyl. Die Reste $H(CH_2)_p—$ und $H(CH_2)_q—$ können zusätzlich Methyl, Ethyl und n-Propyl sein. Beispiele für die $—(CH_2)_r—$Gruppe sind Ethylen, 1,3—Propylen, 1,4—Butylen, 1,5—Pentylen und 1-6 Hexylen. In dieser Gruppe bedeutet r bevorzugt eine Zahl von 2 bis 4, besonders 2 oder 3. Das zweiwertige Metallatom M in Formel I ist bevorzugt Kobalt, Nickel, Kupfer, Palladium und Platin. In einer anderen bevorzugten Ausführungsform steht R für n-Hexyl und $R^1$ für $n—C_6H_{13}O$-.

Ein weiterer Gegenstand der Erfindung ist ein Verfahren zur Herstellung von Metallkomplexen der Formel I, das dadurch gekennzichnet ist, dass man in an sich bekannter Weise ein o-Phenylendiamin der Formel II

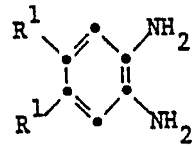

(II)

in Gegenwart eines inerten Lösungsmittels mit einem Metall (II) salz von Eisen, Kobalt, Nickel, Kupfer, Palladium oder Platin und einem Malondialdehyd der Formel III

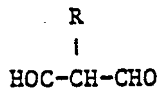
(III)

oder einem Aminoacrolein der Formel IV

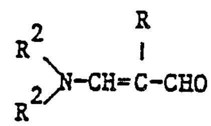
(IV)

wobei R und $R^1$ die in Anspruch 1 angegebene Bedeutung hat und $R^2$ für eine Alkylgruppe steht, umsetzt.

In einer Ausführungsform des Verfahrens geht man zweckmässig so vor, dass man zunächst ein o-Phenylendiamin der Formel II mit einem Metall (II) salz umsetzt und darauf den gebildeten Bis—(o-phenylendiamin)—Komplex mit einem Malondialdehyd der Formel III oder einem Aminoacrolein der Formel IV umsetzt.

In einer weiteren bevorzugten Ausführungsform führt man die Reaktion so durch, dass man zunächst ein o-Phenylendiamin der Formel II mit einem Aminoacrolein der Formel IV umsetzt und anschliessend das gebildete substituierte Dibenzotetraaza — [14] — annulen mit einem Metall (II) salz komplexiert.

Die Reaktion wird vorteilhaft bei Temperaturen von 40 bis 180 °C, besonders 40 bis 150 °C vorgenommen. $R^2$ in Formel IV ist bevorzugt $C_1$—$C_4$—Alkyl, besonders Methyl.

Die Verbindungen der Formeln II, III und IV sind bekannt oder können nach analogen Verfahren hergestellt werden.

Als Metall (II) salze sind z. B. Halogenide wie z. B. Bromide und Chloride, Carbonate, Sulfate, Phosphate, Sulfonate, Fluorsulfate, Nitrate, Cyanide und besonders die Salze von Carbonsäuren, insbesondere die Acetate geeignet.

Die Reaktanden werden bevorzugt in äquimolaren Mengen eingesetzt. Als inerte Lösungsmittel kommen besonders polare aprotische Lösungsmittel in Frage. Beispiele sind besonders Säureamide wie Hexamethylphosphorsäuretriamid, Dimethylformamid, Dimethylacetamid, N-Methylpyrrolidon, sowie Aether wie Dioxan, Ethylenglykoldimethyläther, Diethylenglykoldimethyläther, und halogenierte aromatische Kohlenwasserstoffe wie Chlorbenzol, 1,4-Dichlorbenzol oder 1,2,4-Trichlorbenzol.

Die erfindungsgemässen Metallkomplexe werden nach üblichen Methoden isoliert, z. B. Kristallisation oder Verdampfen des Lösungsmittels. Die Rohprodukte können z. B. durch Umkristallisation, Sublimation oder chromatographische Methoden weiter gereinigt werden. Man erhält einheitliche Produkte hoher Reinheit.

Bei den erfindungsgemässen Metallkomplexen handelt es sich um farbige, z. B. rote, braune oder violette Festkörper in kristalliner Form. Die molaren Extinktionskoeffizienten der im sichtbar liegenden Adsorptionsbanden weisen Werte zwischen etwa 70.000 bis 100.000 auf. Sie sind in organischen Lösungsmitteln löslich, besonders in unpolaren oder schwach polaren Lösungsmitteln.

Viele der bekannten Verbindungen mit flüssigkristallinen Eigenschaften sind farblos. Es wurde gefunden, dass solche farblosen Verbindungen mit den erfindungsgemässen Metallkomplexen mischbar sind und gefärbt werden können, wobei deren flüssigkristalline Eigenschaften praktisch nicht verändert werden, selbst wenn der erfindungsgemässe Metallkomplex selbst nicht flüssigkristallin ist. Ein Beispiel für solche farblosen Verbindungen sind Hexaalkoxytriphenylene (vgl. EP-A-0 047 198 und EP-A-0 030 879).

Ueberraschend wurde gefunden, dass bei den erfindungsgemässen Metallkomplexen selbst trotz der niedrigen zweizähligen Molekülsymmetrie mit wenigen Ausnahmen nach dem Schmelzen diskotische Mesophasen auftreten, die hochviskos sind. Eigene Untersuchungen ergaben, dass diese Eigenschaft nur bei den Metallkomplexen, nicht aber bei metallfreien, entsprechend substituierten Dibenzotetraaza — [14] — annulenen beobachtet wird. Wesentlich für diese Eigenschaft ist auch, dass R in Formel I eine Alkylgruppe ist. Die flüssigkristallinen Eigenschaften werden im wesentlichen durch die Wahl von R und $R^1$ in Formel I bestimmt, hauptsächlich durch unterschiedliche Kettenlängen von R und $R^1$ und die Art der Brückengruppe X. Eine bevorzugte Untergruppe erfindungsgemässer Metallkomplexe sind solche, in denen X in Formel I -O- bedeutet und die Differenz von m-n oder n-m 0 bis 4, besonders 0, 1 oder 2 beträgt.

Die Metallkomplexe mit diskotischen Mesophasen können zur Herstellung von thermooptischen und elektrooptischen Anzeigenelementen verwendet werden, was ein weiterer Gegenstand vorliegender Erfindung ist.

Es ist bekannt, dass Dibenzotetraaza — [14] — annulen Metallkomplexe als Elektronendonatoren zur Herstellung von elektrisch leitenden Chargetransfer-Metallkomplexsalzen verwendet werden können [vgl. Helv. Chim. Acta, Vol. 64, Fasc. 8, S. 2544 bis 2554 (1981)]. Geeignete Elektronenakzeptoren sind z. B. Halogene wie Chlor, Brom und besonders Jod. Auch die erfindungsgemässen Metallkomplexe

können dotiert und in elektrisch leitende Chargetransfer-Komplexsalze überführt werden. Ein weiterer Gegenstand vorliegender Erfindung ist die Verwendung von Metallkomplexen der Formel I als Elektronendonatoren zur Herstellung von organischen elektrischen Leitern, besonders in Form flüssigkristalliner Leiter. Es wurde gefunden, dass die flüssigkristallinen Eigenschaften durch die Dotierung nicht verloren gehen.

Die nachfolgenden Beispiele erläutern die Erfindung näher. Die Phasenumwandlungstemperaturen werden mittels Differential-Scanning-Calorimetrie (DSC) bestimmt. Das Sichtbarspektrum wird in Chloroformlösungen bestimmt.

A) Herstellungsbeispiele

Beispiele 1-33

Die in nachfolgender Tabelle aufgeführten Metallkomplexe werden nach folgenden Verfahren hergestellt:

a) 5 mMol 4,5-disubstituiertes 1,2-Phenylendiamin werden in 25 ml entgastem Dimethylformamid (DMF) gelöst, auf 65 °C erwärmt und 2,5 mMol Metall (II) acetathydrat zugegeben. Nach 45 Minuten Rühren werden 5,5 mMol 2-R-3-dimethylaminoacrolein in 5 ml entgastem DMF gelöst zugetropft. Darauf wird 16 Stunden bei 120 °C gerührt, wobei gleichzeitig langsam Stickstoff durchgeleitet wird, um das gebildete Dimethylamin zu entfernen. Das Reaktionsgemisch wird auf Raumtemperatur abgekühlt und der kristalline Niederschlag abgenutscht, mit $H_2O$ und Ethanol gewaschen und getrocknet. Zur Reinigung wird das Rohprodukt mit kaltem Chloroform gewaschen und anschliessend aus Chloroform umkristallisiert.

b) 6,3 mMol 4,5-Disubstituiertes 1,2-Phenylendiamin werden unter $N_2$-Atmosphäre in 10 ml entgastem DMF gelöst und mit 3,15 mMol Metall (II) acetathydrat versetzt. Man rührt 45 Minuten bei 60 °C und tropft dann eine Lösung von 6,3 mMol 2-R-Malondialdehyd in 5 ml entgastem DMF zu, erwärmt auf 120 °C und rührt 20 Stunden. Nach Abkühlen auf Raumtemperatur wird der kristalline Niederschlag abfiltriert, mit Wasser und Ethanol gewaschen und getrocknet.

In nachfolgender Tabelle bedeutet K gleich kristallin, D gleich diskotisch und I gleich isotrop.

(Siehe Tabelle Seite 5 ff.)

Tabelle

| Beispiel No. (Verfahren a oder b) | M | R | $R^1$ | Sichtbarspektrum $\lambda_{max}$ in nm (log $\varepsilon$) | Phasenübergangstemperatur in °C (Enthalpie in J/g) | |
|---|---|---|---|---|---|---|
| | | | | | K → D | D → I |
| 1 (b) | Ni | $n-C_4H_9$ | $n-OC_4H_9$ | 462 (4,87) | 212 (20,4) | 233 (15,6) |
| 2 (a) | Ni | $n-C_4H_9$ | $n-OC_6H_{13}$ | 462 (4,87) | 155,7 (64) | 175 (5,9) |
| 3 (a) | Ni | $n-C_4H_9$ | $n-OC_8H_{17}$ | 462 (4,86) | 146,5 (81) | 150 (6,0) |
| 4 (a) | Ni | $n-C_4H_9$ | $n-OC_{14}H_{29}$ | 462 (4,86) | 136,2 | keine Mesophase |
| 5 (a) | Ni | $n-C_6H_{13}$ | $n-OC_6H_{13}$ | 462 (4,86) | 131 | 204 (9,3) |
| 6 (a) | Ni | $n-C_6H_{13}$ | $n-OC_8H_{17}$ | 462 (4,86) | 132 | 171 (7,3) |
| 7 (a) | Ni | $n-C_6H_{13}$ | $n-OC_{12}H_{25}$ | 462 (4,86) | 132 (136) | 126(3,4), I→D 122 monotrop |
| 8 (b) | Ni | $n-C_8H_{17}$ | $n-OC_6H_{13}$ | 462 (4,87) | 140 | 207 (10,4) |
| 9 (a) | Ni | $n-C_8H_{17}$ | $n-OC_8H_{17}$ | 460 (4,87) | 137 | 184 (7,8) |
| 10 (a) | Ni | $n-C_8H_{17}$ | $n-C_9H_{19}$ | 454 (4,88) | 126 (69) | 90, I→D 88,4 monotrop |
| 11 (a) | Ni | $n-C_8H_{17}$ | $n-OC_{10}H_{21}$ | 462 (4,86) | 136 | 158 (5,8) |
| 12 (b) | Ni | $n-C_8H_{17}$ | $n-OC_{12}H_{25}$ | 461 (4,85) | 128 (90) | keine Mesophase |
| 13 (a) | Ni | $n-C_8H_{17}$ | $-COOn-C_8H_{17}$ | 457 (4,90) | 255 (13) | 255(9), I→D 249 monotrop |
| 14 (b) | Ni | $n-C_8H_{17}$ | $n-OC_{14}H_{29}$ | 461 (4,85) | 131 (56) | keine Mesophase |
| 15 (a) | Ni | $n-C_{10}H_{21}$ | $n-OC_8H_{17}$ | 462 (4,86) | 118 (77) | 182 (8,3) |
| 16 (a) | Ni | $n-C_{10}H_{21}$ | $n-C_9H_{19}$ | 452 (4,88) | 116 (70) | 94,5 (3,3) I→D 91,4 monotrop |
| 17 (a) | Ni | $n-C_{10}H_{21}$ | $n-OC_{10}H_{21}$ | 462 (4,86) | 119 (58) | 163 (6,3) |

Tabelle (Fortsetzung)

| Beispiel No. (Verfahren a oder b) | M | R | $R^1$ | Sichtbarspektrum $\lambda_{max}$ in nm (log ε) | Phasenübergangstemperatur in °C (Enthalpie in J/g) | |
|---|---|---|---|---|---|---|
| | | | | | K ⟶ D | D ⟶ I |
| 18 (a) | Ni | $n-C_{10}H_{21}$ | $n-OC_{12}H_{25}$ | 463 (4,87) | 119 (81) | 139 (3,5) |
| 19 (a) | Ni | $n-C_{10}H_{21}$ | $n-OC_{14}H_{29}$ | 462 (4,86) | 120 | 123 (3,7) |
| 20 (a) | Ni | $n-C_{10}H_{21}$ | $-COO-n-C_8H_{17}$ | 487 (4,89) | 254(14) | 254(9), I ⟶ D 250 monotrop |
| 21 (a) | Ni | $n-C_{15}H_{31}$ | $n-OC_{14}H_{29}$ | 462 (4,85) | 104 (111) | 119 (~ 2) |
| 22 (a) | Pd | $n-C_6H_{13}$ | $n-OC_6H_{13}$ | 464 (5,07) | 136 (92) | 239 (17) |
| 23 (a) | Pt | $n-C_6H_{13}$ | $n-OC_6H_{13}$ | 444 (5,04) | 153 | 213 (10,7) |
| 24 (a) | Co | $n-C_6H_{13}$ | $n-OC_6H_{13}$ | 418 (4,70) | 128 (92) | 216 (12,4) |
| 25 (a) | Co | $n-C_{10}H_{21}$ | $n-OC_8H_{17}$ | 418 (4,74) | 115 (79) | 193 (9,6) |
| 26 (a) | Co | $n-C_{15}H_{31}$ | $n-OC_{14}H_{29}$ | 418 (4,72) | 100 (80) | 140 (5,4) |
| 27 (a) | Cu | $n-C_6H_{13}$ | $n-OC_6H_{13}$ | 432 (5,02) | 123 (93) | 242 (12,3) |
| 28 (a) | Cu | $n-C_8H_{17}$ | $n-OC_8H_{17}$ | 433 (5,00) | 133 (91) | 219 (6,5) |
| 29 (a) | Cu | $n-C_{10}H_{21}$ | $n-OC_{14}H_{29}$ | 430 (5,01) | 114 (87) | 163 (6,2) |
| 30 (a) | Ni | $n-C_6H_{13}$ | $-OCH_2CH_2OC_2H_5$ | 461 (4,88) | 148 (125) | 206 (12) |
| 31 (a) | Ni | $n-C_8H_{17}$ | $-OCH_2CH_2OC_2H_5$ | 461 (4,88) | 157 (83) | 189 (8) |
| 32 (a) | Ni | $n-C_8H_{17}$ | $-O(CH_2)_2O(CH_2)_2OCH_3$ | 461 (4,90) | 121 (83) | 112 (2,2) monotrop |
| 33 (a) | Ni | $n-C_{10}H_{21}$ | $-O(CH_2)_2O(CH_2)_2OCH_3$ | 461 (4,86) | 114 (77) | keine Mesophase |

B) Anwendungsbeispiele

Beispiel 30

Flüssigkristalliner elektrischer Leiter 100 mg Metallkomplex No. 5 werden in 25 ml n-Heptan (Rückfluss) gelöst und mit 3,5 mg Jod in 1 ml Trichlorbenzol versetzt. Nach Abkühlen auf Raumtemperatur werden die violetten Kristalle (74 mg) abfiltriert. Das Verhältnis von Metallkomplex zu Jod beträgt 1 : 0,07. Die elektrische Leitfähigkeit beträgt $10^{-7}$ S $cm^{-1}$. Die Phasenumwandlungstemperaturen sind im Vergleich zum nichtjodierten Metallkomplex unverändert. Im flüssigkristallinen Bereich bei 165 °C beträgt die Leitfähigkeit $4.10^{-9}$ S $cm^{-1}$.

Beispiel 31

Färben von flüssigkristallinen Verbindungen.

a) 9 mg des Metallkomplexes No. 20 wird in 50 mg Rufigallol-hexa-n-octanoat (A) gelöst. Die orangerote Mischung weist unveränderte Phasenumwandlungstemperaturen auf im Vergleich zum ungefärbten A : K → D : 108 °C, D → I : 128 °C.

b) n-Hexapentyloxytriphenylen wird mit dem Metallkomplex No. 13 im Gewichtsverhältnis von 9 : 1 vermischt. Beim Erwärmen bildet sich eine orangerote Lösung mit flüssigkristallinen Eigenschaften im Bereich von 65 °C bis 118 °C. Vergleichsweise weist das reine n-Hexapentyloxytriphenylen einen Bereich von 69 °C bis 122 °C auf.

c) Verfährt man gemäss b), aber mit dem Metallkomplex No. 27, so erhält man beim Erwärmen eine goldgelbe Lösung mit einem flüssigkristallinen stabilen Bereich von 66 bis 122 °C.

**Patentansprüche**

1. Substituierte Dibenzotetraaza — [14] — annulen-metallkomplexe der Formel I

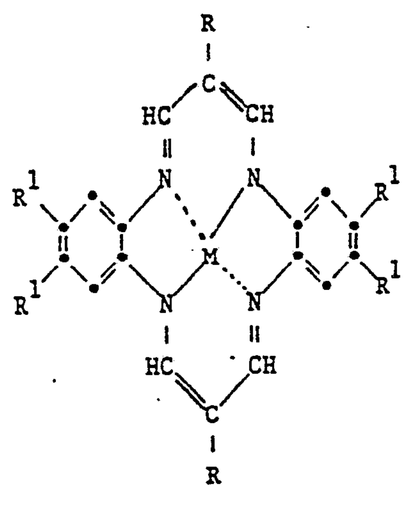

(I)

worin M für ein zweiwertiges Metallatom aus der Gruppe Eisen, Kobalt, Nickel, Kupfer, Palladium und Platin steht, R den Rest $H(CH_2)_m$ — bedeutet, worin m eine ganze Zahl von 4 bis 18 bedeutet, und $R^1$ für den Rest $H(CH_2)_n$—X—, $H(CH_2)_p$—Y—$CH_2$—Z— oder $H(CH_2)_q$—[—O—$(CH_2)_r$—]—$_s$Z— stehen, worin n eine ganze Zahl von 4 bis 18 bedeutet und X für eine direkte Bindung, -S-, -SO-, $SO_2$-, -O-, -C(O)O- oder -O(O)C- steht, Y und Z unabhängig -O- oder -S- bedeuten, p für eine ganze Zahl von 1 bis 15 steht, r für eine ganze Zahl von 2 bis 6, s für eine ganze Zahl von 1 bis 6 und q für eine Zahl von 1 bis 19 — (r + 1) · s stehen.

2. Metallkomplexe gemäss Anspruch 1, dadurch gekennzeichnet, dass m und n eine Zahl von 6 bis 14 und p eine ganze Zahl von 3 bis 11 und die Gruppe $H(CH_2)_q$—[—O — $(CH)_r$—]—$_s$Z- 6 bis 14 Kettenglieder aufweist.

3. Metallkomplexe gemäss Anspruch 1, dadurch gekennzeichnet, dass X für eine direkte Bindung, -S- und besonders -O- steht.

4. Metallkomplexe gemäss Anspruch 1, dadurch gekennzeichnet, dass M als zweiwertiges Metallatom Kobalt, Nickel, Kupfer, Palladium oder Platin ist.

5. Metallkomplex gemäss Anspruch 1, dadurch gekennzeichnet, dass der Unterschied in der Kettenlänge der R- und $R^1$-Gruppen höchstens 6 Kettenglieder beträgt.

6. Metallkomplexe gemäss Anspruch 1, dadurch gekennzeichnet, dass in Formel I R für n-Hexyl und $R^1$ für n-Hexoxy stehen.

7. Metallkomplexe gemäss Anspruch 1, dadurch gekennzeichnet, dass X für -O- steht und die Differenz von m — n oder n — m von 0-4, bevorzugt 0, 1 oder 2 beträgt.

8. Verfahren zur Herstellung von Metallkomplexen der Formel I gemäss Anspruch 1, dadurch gekennzeichnet, dass man ein o-Phenylendiamin der Formel II

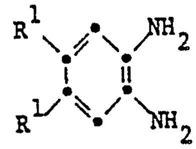

(II)

in Gegenwart eines inerten Lösungsmittels mit einem Metall (II) salz von Eisen, Kobalt, Nickel, Kupfer, Palladium oder Platin und einem Malondialdehyd der Formel III

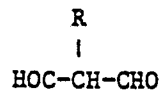

(III)

oder einem Aminoacrolein der Formel IV

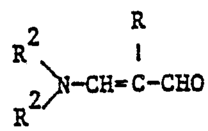

(IV)

wobei R und $R^1$ die in Anspruch 1 angegebene Bedeutung haben und $R^2$ für eine Alkylgruppe steht, umsetzt.

9. Verfahren gemäss Anspruch 8, dadurch gekennzeichnet, dass man (a) zunächst ein o-Phenylendiamin der Formel II mit einem Metall (II) salz umsetzt und darauf den gebildeten Bis — (o-phenylendiamin)-Komplex mit einem Malondialdehyd der Formel III oder einem Aminoacrolein der Formel IV umsetzt, oder dass man (b) zunächst ein o-Phenylendiamin der Formel II mit einem Aminoacrolein der Formel IV umsetzt und anschliessend das gebildete substituierte Dibenzotetraaza — [14] — annulen mit einem Metall (II) salz komplexiert.

10. Verfahren gemäss Anspruch 8, dadurch gekennzeichnet, dass die Reaktion bei einer Temperatur von 40 bis 180 °C vorgenommen wird.

11. Verwendung von Metallkomplexen der Formel I gemäss Anspruch 1 zur Herstellung von elektrooptischen und thermooptischen Anzeigenelementen.

12. Verwendung von Metallkomplexen der Formel I gemäss Anspruch 1 als Elektronendonatoren zur Herstellung von organischen elektrischen Leitern, besonderes in Form flüssigkristalliner Leiter.

## Claims

1. A substituted dibenzotetraaza — [14] — annulene metal complex of the formula I

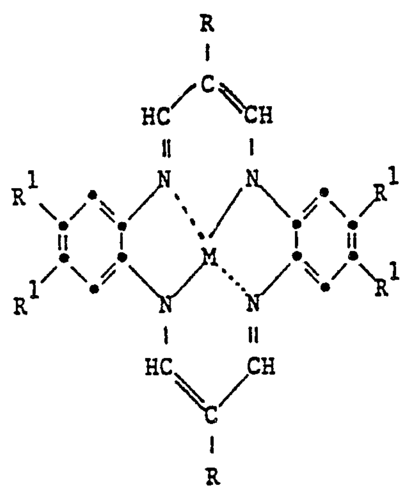

(I)

in which M is a bivalent metal atom selected from the group iron, cobalt, nickel, copper, palladium and platinum, R is the radical $H(CH_2)_m$, in which m is an integer from 4 to 18, and $R^1$ is the radical $H(CH_2)_n$-X-, $H(CH_2)_p$-Y-$CH_2$-Z or $H(CH_2)-_q-[-O-CH_2)_r-]-_sZ$-, in which n is an integer from 4 to 18, X is a direct bond, -S-, -SO-, -$SO_2$-, -O-, -C(O)O- or O(O)C-, Y and Z are each independently -O- or -S-, p is an integer

from 1 to 15, r is an integer from 2 to 6, s is an integer from 1 to 6, and q is an integer from 1 to 19 — (r + 1) · s.

2. A metal complex according to claim 1, wherein m and n are numbers from 6 to 14 and p is an integer from 3 to 11 and the $H(CH_2)_q—[—O—(CH)_r—]—_sZ$ — group has 6 to 14 chain members.

3. A metal complex according to claim 1, wherein X is a direct bond, -S- and, in particular, -O-.

4. A metal complex according to claim 1, wherein M as a bivalent metal atom is cobalt, nickel, copper, palladium or platinum.

5. A metal complex according to claim 1, wherein the difference in the chain length of the R and $R^1$ groups is at most 6 chain members.

6. A metal complex according to claim 1, wherein in formula I R is n-hexyl and $R^1$ is n-hexoxy.

7. A metal complex according to claim 1, wherein X is -O- and the difference of m — n or n — m is from 0 to 4, preferably 0, 1 or 2.

8. A process for the preparation of a metal complex of the formula I according to claim 1, which comprises reacting an o-phenylenediamine of the formula II

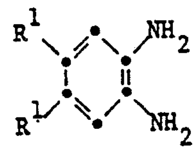

(II)

with a metal (II) salt of iron, cobalt, nickel, copper, palladium or platinum and a malonic dialdehyde of the formula III

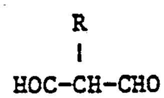

(III)

or an aminoacrolein of the formula IV

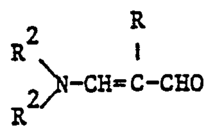

(IV)

where R and $R^1$ are as defined in claim 1 and $R^2$ is an alkyl group, in the presence of an inert solvent.

9. A process according to claim 8, which comprises (a) initially reacting an o-phenylenediamine of the formula II with a metal (II) salt and then reacting the resultant bis (o-phenylenediamine) complex with a malonic dialdehyde of the formula III or an aminoacrolein of the formula IV, or (b) initially reacting an o-phenylenediamine of the formula II with an aminoacrolein of the formula IV and then complexing the resultant substituted dibenzotetraaza — [14] — annulene with a metal (II) salt.

10. A process according to claim 8, wherein the reaction is carried out at a temperature from 40 to 180 °C.

11. Use of a metal complex of the formula I according to claim 1 for the preparation of electro-optical and thermo-optical display elements.

12. Use of a metal complex of the formula I according to claim 1 as electron donor for the praparation of organic electrical conductors, in particular in the form of liquid crystalline conductors.

**Revendications**

1. Complexes métalliques de dibenzo-tétraza — [14] — annulènes substitués répondant à la formule I :

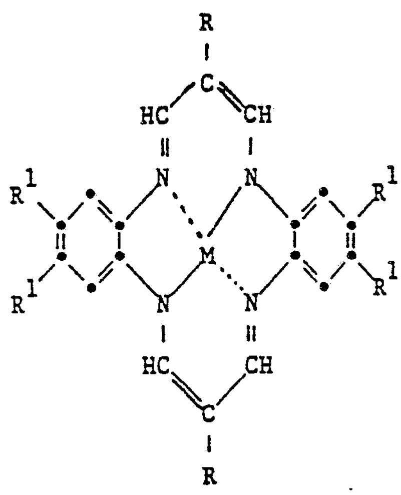

(I)

dans laquelle

M représente un atome de métal bivalent pris dans l'ensemble constitué par le fer, le cobalt, le nickel, le cuivre, le palladium et le platine,

R représente un radical $H(CH_2)_m$- dont l'indice m désigne un nombre entier de 4 à 18, et

$R^1$ représente un radical répondant à l'une des formules : $H(CH_2)_n$—X—, $H(CH_2)_p$—Y—$CH_2$—Z— et $H(CH_2)_q$—[—O—$(CH_2)_r$—]—$_s$ Z dans lesquelles n désigne un nombre entier de 4 à 18, X représente une liaison directe -S-, -SO-, -$SO_2$-, -O-, -C(O)O ou -O(O)C-, Y et Z représentent chacun, indépendamment l'un de l'autre, -O- ou -S-, p désigne un nombre entier de 1 à 15, r un nombre entier de 2 à 6, s un nombre entier de 1 à 6, et q un nombre entier de 1 à [19 — (r + 1).s].

2. Complexes métalliques selon la revendication 1 caractérisés en ce que m et n représentent chacun un nombre de 6 à 14, p représente un nombre entier de 3 à 11, et le radical $H(CH_2)_q$—[—O—$(CH_2)_r$—]—$_s$—Z— contient, dans sa chaîne, de 6 à 14 maillons.

3. Complexes métalliques selon la revendication 1 caractérisés en ce que X représente une liaison directe, -S- ou, plus particulièrement, -O-.

4. Complexes métalliques selon la revendication 1 caractérisés en ce que M, en tant qu'atome de métal bivalent, représente un atome de cobalt, de nickel, de cuivre, de palladium ou de platine.

5. Complexe métallique selon la revendication 1 caractérisé en ce que la différence entre les longueurs de chaînes des radicaux R et $R^1$ est d'au plus 6 maillons.

6. Complexes métalliques selon la revendication 1 caractérisés en ce que, dans la formule I, R représente un radical n-hexyle et $R^1$ un radical n-hexyloxy.

7. Complexes métalliques selon la revendication 1 caractérisés en ce que X représente -O- et en ce que la différence (m — n) ou (n — m) est égale à un nombre de 0 à 4, de préférence est égale à 0 à 1 ou à 2.

8. Procédé de préparation de complexes métalliques de formule 1 selon la revendication 1, procédé caractérisé en ce qu'on fait réagir une o-phénylène-diamine de formule II :

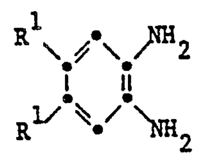

(II)

en présence d'un solvant inerte, avec un sel de métal (II) qui est un sel de fer, de cobalt, de nickel, de cuivre, de palladium ou de platine, et avec un malonaldéhyde de formule III :

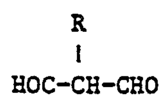

(III)

ou une amino-acroléine de formule IV :

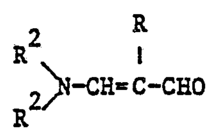

(IV)

formules dans lesquelles R et $R^1$ ont les significations données à la revendication 1 et $R^2$ représente un radical alkyle.

9. Procédé selon la revendication 8 caractérisé en ce que a) on fait d'abord réagir une o-phénylène-diamine de formule II avec un sel de métal (II), puis on fait réagir le complexe de bis-(o-phénylène-diamine) formé avec un malonaldéhyde de formule III ou une amino-acroléine de formule IV ; ou b) on fait d'abord réagir une o-phénylène-diamine de formule II avec une amino-acroléine de formule IV, puis on complexe par un sel de métal (II) le dibenzo-tétraza — [14] — annulène substitué formé.

10. Procédé selon la revendication 8 caractérisé en ce qu'on effectue la réaction à une température de 40 à 180 °C.

11. Application de complexes métalliques de formule I selon la revendication 1 à la fabrication d'éléments d'affichage électro-optique et thermo-optique.

12. Application de complexes métalliques de formule I selon la revendication 1 comme donneurs d'électrons pour la fabrication de conducteurs électriques organiques, en particulier de conducteurs à cristaux liquides.